# EUROPEAN PATENT APPLICATION

(11) **EP 3 090 763 A1**
(43) Date of publication of application: **09.11.2016**
(21) Application number: 15450020.1
(22) Date of filing: 04.05.2015
(51) Int. Cl.: A61L 15/34, A61L 15/40, A61L 15/42, A61L 15/44, A61L 15/46

(54) **WOUND COVERING MATERIAL**

(71) Applicant: IME GmbH, 9020 Klagenfurt am Wörthersee (AT)
(72) Inventor: Likon, Marko, SI-6280 Ankaran (SI); Remskar, Maja, SI-1215 Medvode (SI)
(74) Representative: Gibler & Poth Patentanwälte KG

(57) **Abstract**

A wound covering material (1) for directly contacting a wound (2) comprising.a fabric (3) made of fibers (4) is proposed, wherein the fibers (4) are natural hydrophobic hollow fibers (4). Further a method for producing a wound covering material (1) is proposed.

## Description

The present invention relates to wound covering material for directly contacting a wound.

Open and/or chronic wounds typically secrete exudates during the healing process. Until recently it was generally desirable to remove these wound exudates from the wound vicinity to minimize bacterial growth which can cause infection and delay healing. Excessive exposure to wound exudates can result in maceration of the skin surrounding the wound. Wound exudates are known to contain tissue degrading enzymes that can impede wound healing. It is generally known to cope with exudates of large open chronic wounds by packing them with an absorbent packing such as gauze. Gauze packing cells dead space in the wound and absorbs exudates released by the tissue over time.

Disadvantageously, the absorbent gauze must be replaced periodically as it absorbs a larger volume of exudates and becomes saturated. US 7,754,937 B2 describes the production of wound packing materials with improved sucking capability.

However, the removal of exudates drains the wound causes formation of scabs which inhibits the wounds healings. For this reason it was tried to develop hydrophobic wound covering materials which bound pathogenic bacteria which produced enzymes and toxic substances but still offers the lubrication of the wounds. US 6,160,196 A shows a wound covering which is characterized in that it is made of a hydrophobic bacteria-adsorbing material which comprises an antimicrobial active compound which is not released into the wound and exploited synergistic effect of hydrophobicity of fibers and biocidal spots placed onto carrying fibers. The bacteria are simply removed from wound with removing the wound covering. Described solution do not offers wound breathing due to material hydrophobicity and it is based on polymers production. Production of these materials presents environmental burden during production as well at disposal after usage.

Antimicrobial treatment of wounds or preventive treatment of wounds to protect them against infections by pathogenic bacteria have been known for a long time. The use of bactericidal agents as oxidizers, disinfectants or antiseptic chemicals may be mentioned here as example. If bactericidal agents are employed in an extra pure form, in solution, as an ointment or in another presentation form, the action mechanism is always comparable. Microorganisms such as bacteria are destroyed but dead cells remain in the wound. Such treated wounds cannot be cleaned to remove the active compound reliably after the application, since it spreads onto entire affected area. Precise dosage of the bactericidal agents onto the wound is difficult and usually these active compounds occur freely in the wound and they can also attack and destroy cells and substances in the wound fluid which promote wound healing, for example damage to healing promoting protein substances by iodine or peroxides. One of the next steps in wound treatment was to cover wounds with sterilized or antibacterial treated wound coverings to prevent further infections. These wound coverings can be prepared, for example, by treatment of carriers such as woven fabrics, nonwovens, gauze and the like with or without active compounds. By covering wounds with these materials which do not release the active compound, however, served only as protective layer which presents merely a barrier for microorganisms which are prevented from penetrating into the wound from the outside by being destroyed on contact with the material. It therefore cannot be regarded as treatment of an infected wound. However, if the active compound is released from anti microbially treated wound coverings into the wound, the same disadvantages arise as with application in an extra pure form, as a solution, as an ointment or in another presentation form because the bacteria remain in the wound, no reliable cleaning of the wound is possible and troublesome effects may occur.

Use of hydrophobized materials enabled adsorption of bacteria onto a wound covering what was confirmed with previous research which proved adsorption of pathogenic bacteria onto hydrophobic surfaces or onto hydrophobized fibers. The bacteria are then withdrawn from the wound by removal of the wound covering. A decisive disadvantage here is that, in contrast to the customary treatment methods mentioned above, bacteria and microorganisms are not destroyed.

In US 6,160,196 A a wound covering is described which is characterized in that it is made of a hydrophobic bacteria-adsorbing material which comprises an antimicrobial active compound which is not released into the wound and exploited synergistic effect of hydrophobicity of supporting fibers and biocidal anchors placed onto the surface of carrying fibers. The main shortcomings of this solution is preparation of material which includes combination of hydrophobic fibers and hydrophilic fibers, carriers of antimicrobial active compounds. Hydrophilic part remove moisture from the wound and do not allow active healing and formation of the scab.

All mentioned materials are synthetic and are chemically treated. They presents environmental burden during production as well at disposal after usage.

It is therefore an object of the invention to provide a wound covering material which can be employed for treatment of infected wounds, or for preventive protection against wound infections, with prolonged action, which supports wound healing, is easy to produce, ecological and therefore easy to dispose.

According to the present invention, there is provided a wound covering material as set out in claim 1.

Advantageously, the hydrophobic structure controls evaporation of moisture from the wound and prevents drainage of the wound and consequentially dying of the cells but on the same time enables aeration of the affected area. Hydrophobic surface attracting the pathogenic bacteria from the wound exudates and physically bind it onto hydrophobic surface. Furthermore the wound covering material is easy to produce and environment-friendly. Therefore their disposal after usage is no environmental burden.

Furthermore it is an object of the invention to provide a method for producing a wound covering material.

According to the present invention, there is further provided method for producing a wound covering material as set out in claim 12.

Additional advantageous features are set out in the dependent claims.

Reference is hereby made expressly to the wording of the claims, by means of which the claims shall be regarded as inserted at this point into the description by reference and shall be regarded as being reproduced literally.

The invention will be explained in closer detail by reference to the enclosed drawings which merely show preferred embodiments by way of example, wherein:
Fig. 1 shows a SEM image of the poplar seed fibers;
Fig. 2 shows a SEM image of the poplar seed fibers wall with a hydrophobic wax layer;
Fig. 3 shows a SEM image of a preferred embodiment, and
Fig. 4 a sketch of a preferred embodiment of the wound covering material used on a wound.

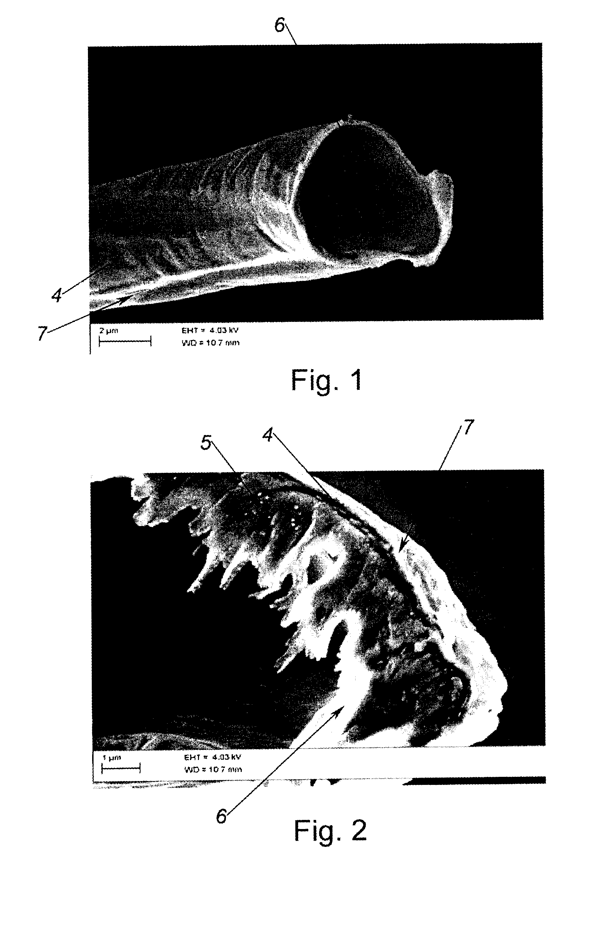

Figs. 1 to 4 show at least part of a preferred embodiment of a wound covering material 1 for directly contacting a wound 2 comprising a fabric 3 made of fibers 4, wherein the fibers 4 are natural hydrophobic hollow fibers 4. Natural hydrophobic hollow fibers 4 comprises natural grown fibers with hydrophobic hollow morphological structure.

Advantageously, the hydrophobic structure controls evaporation of moisture from the wound 2 and prevents drainage of the wound 2 and consequentially dying of the cells but on the same time enables aeration of the affected area. Hydrophobic surface attracting the pathogenic bacteria 9 from the wound exudates 10 and physically bind it onto hydrophobic surface. Furthermore the wound covering material 1 is easy to produce and environment-friendly. Therefore their disposal after usage is no environmental burden.

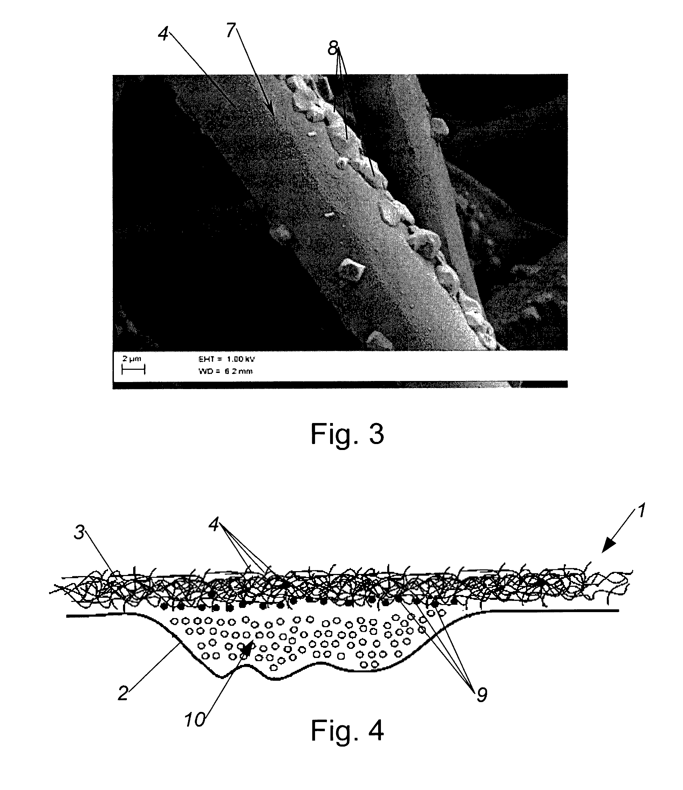

In Fig. 4 the pathogenic bacteria 9 is represented by dots and the wound exudates 10 are represented by hollow circles.

Further the use of this wound covering material 1 for covering a, particularly infected, wound 2 is proposed.

It can be provided according to a further development of the invention that the fabric 3 is a nonwoven fabric, woven fabric, knitted fabric or net.

It can be provided according to a further development of the invention that the natural hydrophobic hollow fibers 4 are poplar seed fibers 4 and/or kapok fibers 4.

It can be provided according to a further development of the invention that the natural hydrophobic hollow fibers 4 are covered with natural waxy layers 5

The poplar seed fibers 4 and kapok fibers 4 are natural fibers 4 with the common characteristic that micro morphological structure of theses fibers 4 are hollow tube. The tube walls are chemically compose from lignocellulosic matrix and the wall surface 6,7 are layered with hydrophobic natural wax 5, which contain fatty acids compounds and traces of parabene compounds. The wax layer 5 itself act bactericidal onto attached bacteria 9. The fibers 4 express high hydrophobicity and more than 77 % of fibers 4 presents empty lumen.

Poplar and similar fibers 4 generates from Poplar and aspen trees. Latest are angiosperm trees genus Populus which originates from Salicaceae plant family. These trees of different subgenres are widespread in western and central Europe and most parts of North America. The fibers 4 derived from the seeds of Populus trees which are, by their nature, hydrophobic/oleophilic micro tubes with 8.74 ± 5.75 µm of outer diameter, 8.24 ± 4.79 µm of internal diameter and 4 ± 1 mm in length. Micro-tubes have an average wall thickness of 500 ± 30 nm and are composed of 33-37 % of cellulose, 19-22 % of hemicelluloses, manly pentosans, 10-12 % of lignin and 1 -2 % of inorganic substances. Fibers 4 have a high acetyl groups content, approximately 19-22 %, suggesting a degree of acetylation of the saccharides from 1.3 to 1.4 of acetyl groups per monosaccharide unit. Bulk density of the fibers 4 is 0.0036 g/cm3 as 89 vol. % of the fiber 4 is empty lumen. This lumen tends to be filled with hydrophobic/oleophilic substance when the fiber 4 comes into contact with this substance. Hydrophobicity of the fibers 4 is increased due to a coat of fiber 4 surface 6,7 with natural waxes 5. Due to their low ratio between length and external diameter, fibers 4 express improved resistance to gel blockage and higher mechanical resistance against collapse. An SEM image of a poplar seed fiber 4 is provided in Fig. 1. An SEM image of a poplar seed fiber 4 with a natural waxy layer 5 is provided in Fig. 2.

The SEM images of Fig. 1 to 3 further provides a scale bar, the energy of the used electron beam EHT and the used working distance WD of the respective image.

Kapok fibers 4 generates the Ceiba pentandra (L.) Gaertn. This tree belongs to the family of the Malvaceae plants and has origin in South America but it can be found in tropical areas of Southeast Asia, Sri Lanka and other parts of eastern Asia and Africa. Kapok fibers 4 are natural hydrophobic/oleophilic micro tubes with 25 ± 5 mm in length, outside diameter of 16.5 ± 2.4 µm and with an average wall thickness of 2.0 µm. Tube walls are composed of cellulose, hemicelluloses and lignin. Bulk density of kapok fibers 4 is 0.0013 g/cm3 as 77 vol. % of the fibers 4 is empty lumen. Hydrophobicity is enhanced due to acetylation of the wall saccharides and due to the coating of the tube wall surface 6,7 with natural waxes 5. This lumen tends to be filled with hydrophilic/oleophilic substance when fibers 4 come to contact with it. The disadvantage of kapok fibers 4 comparing to poplar fibers 4 is reflected in their mechanical instability due to the high ratio of fiber 4 length versus their external diameter. This results in decrease of the absorption capacity with increasing of package density.

Poplar seed fibers 4 and kapok fibers 4 express high hydrophobicity measured through contact angle related to water. Kapok fibers 4 have contact angle 117° meanwhile poplar seed fibers 4 expressed its super hydrophobicity with contact angle over 150°.

Kapok fibers 4 and poplar seed fibers 4 have tubular, especially hollow, micro/nano structure where 77 % to 89 % of its volume presents empty lumen.

The nonwoven fabric 3 produced from kapok fibers 4 and poplar seed fibers 4 forms three dimensional structures on micro/nano scale. Such a three dimensional structure can be used as an unwoven fabric 3. The hydrophobic structure controls evaporation of moisture from the wound 2 and prevents drainage of the wound 2 and dying of the bioactive cells in the wound 2 but on the same time enables aeration of the affected area. Hydrophobic surface attracting the pathogenic bacteria 9 and bind it onto hydrophobic surface. The hydrophobic waxy layer 5 contains traces of acetylated fatty acids and parabene compounds which acts antibacterial itself. Such wound covering material 1 enables controlled evaporation and aerobic condition on the wound 2 surface and consequentially production of wound covering materials which act on longer period.

Further it can be provided that the fibers 4 are formed to threads to make a woven fabric, knitted fabric or net.

It is especially preferably provided that the natural hydrophobic hollow fibers 4 comprises an inner wall surface 6 and a outer wall surface 7, and that antimicrobial nanoparticles 8 are attached to the inner wall surface 6 and/or a outer wall surface 7. Preferably these antimicrobial nanoparticles 8 can be antibacterial nanoparticles 8. The attached antimicrobial nanoparticles 8 additionally deactivate the bacteria 9 which remains attached onto the wound covering material 1 and prevent releases of deactivated bacteria 9 back into the open wound 2.

It can be further provided that the antimicrobial nanoparticles 8 are metal oxides, in particular MoO₃, ZnO or MgO, or nano silver.

The outer wall surface 7 and inner wall surface 6 of tube walls can be covered with nano layers of highly hydrophobic natural wax 5 contains traces of acetylated fatty acids and parabene compounds and represents anchorages for antimicrobial nanoparticles 8.

It can be further provided that the antimicrobial nanoparticles 8 have a maximal outer diameter of 850 nm.

Further the antimicrobial nanoparticles 8 can be permanently fixed onto the outer wall surface 7 and/or inner wall surface 6 of the tubular structure of the fibers 4.

Fig. 3 shows a SEM image of poplar seed fibers 4 with several MoO₃ nanoparticles 8 attached to their outer wall surfaces 7.

Further a method for producing a wound covering material 1 is provided, which method comprises the steps:
- providing a mixture of raw natural hydrophobic hollow fibers 4 and a liquid solution,
- evenly distributing the natural hydrophobic hollow fibers 4 in the mixture,
- applying the mixture on a mechanical support, and
- drying the mixture.

Furthermore it can be preferably provided wherein a antimicrobial agent is added to the liquid solution or mixture, which antimicrobial agent forms antimicrobial nanoparticles 8 on inner wall surfaces 6 and/or outer wall surfaces 7 of the fibers 4 during the drying.

According to a preferred embodiment of the method the raw fibers 4 are immersed into slurry of antimicrobial nanoparticles 9 and nonpolar to semi polar liquids or/and their mixtures. The slurry are mixed until antimicrobial nanoparticles 9 are dissolved, for example metal oxide nanoparticles 9 as for example MoO₃, ZnO and MgO, or completely dispersed, for example nano silver. The conditions for preparation depends from the nature of solvent or/and dispersion liquid, and nature of solute or/and antimicrobial nanoparticles 9. The mass quantity of antimicrobial nanoparticles 9 in the slurry depends on their solubility or dispersibility in the liquid phase. The raw fibers 4 are immersed into prepared solution or dispersion in the ratio that all fibers4 are completely soaked and covered with solution or dispersion of antimicrobial nanoparticles 9. The mixture of raw fibers 4 and antimicrobial nanoparticles 9 are lightly mixed until the solution or dispersion is evenly distributed through the fibers 4.

Homogenous mixture of fibers 4 and nanoparticles 9 are transferred onto perforated belt as continuous ribbon with the height at least 5 mm. The remained solution are sucked out from the fibers 4 with the vacuum system located under the perforated belt on the way that fibers 4 are compacted into the fabrics 3 on the upper side of the perforated belt.

Prepared fabrics 3 are dried from 30°C to 50°C until constant weight but not shorter than 24 hours. During the drying process the liquid phase evaporates, super saturation of the antimicrobial agent on the fibers 4 surface occurs 6,7 and nanoparticles 9 are formed on the outer wall surface 7 and inner wall surface 6 of the hollow fibers 4. During evaporation process fibers 4 formed interlaced three dimensional net bound by physical forces.

The prepared fabrics 3 are removed from the belt as ribbon or sheets and carefully cutted into the prescribed dimensions.

The method will be described more specifically with reference to working example, which is cited solely for illustration and are not limitative of the invention in any sense.

### EXAMPLE 1

In the 8 mL of the 2.2 wt. % of polyethylene oxide (average Mw = 900,000) in distilled water 10 mg of MoO₃ nanowires were added, with 5.7 wt. % calculated on the mass of polyethylene oxide. Mixture were mixed with the magnetic stirrer 30 minutes until MoO₃ nanowires were equally distributed over the whole volume of the mixture. The solubility of the MoO₃ in water is 1 mg/l. In the case of working example the weight concentration is 1.25 mg MoO₃ per liter of dissolution and after 30 minutes all MoO₃ nanowires have been almost completely dissolved. The 31 mg of raw poplar seed fibers 4 were added into prepared solution, with 17.6 wt. % calculated on the mass of polyethylene oxide. The mixture was shook for 5 minutes by hand due to the equal distribution of fibers 4 over whole mixture. Prepared mixture was left to stay at room temperature for 24 hours for additional homogenization. After homogenization the mixture were evenly applied on piece of cotton fabrics or piece of polyvinylden fluoride which served for mechanical support for active mixture of poplar seed fibers 4, MoO₃ nanoparticles 9 and polyethylene oxide. The mixture was dried for 24 hours at 30°C to prepare final product. A sample of the final product is shown in Fig. 2.

### EXAMPLE 2

A silver sol having a particle size of 150 nm or less, which had been prepared in an aqueous solution according to US 7,135,195 B2, was added to a distilled water detergent contains anionic surfactant and a non-ionic surfactant. Here, the silver sol has a concentration of 100,000 ppm. 1 g of the silver sol was added to 10 mL of distilled water and mixed on the magnetic stirrer that the concentration of silver gel in distilled water was 1 mg/L. The 31 mg of raw poplar seed fibers 4 were added into prepared solution. The mixture was shook for 5 minutes by hand due to the equal distribution of fibers 4 over whole mixture. Prepared mixture was left to stay at room temperature for 24 hours for additional homogenization. After homogenization the mixture were evenly applied on piece of cotton fabrics or piece of polyvinylden fluoride which served for mechanical support for active mixture of poplar seed fibers 4, silver nanoparticles 9 and surfactants. The mixture was dried for 24 hours at 50°C to prepare final product.

Furthermore a wound covering comprising the wound covering material 1 is proposed. Such a wound covering is also known as dressing or medical dressing. The wound covering can comprise the wound covering material 1 itself, which is supposed to directly contact the wound, and a carrier material, which is supposed to support and fixate the wound covering material 1 in respect to the wound 2.

It can be provided that a carrier material comprises a first side with a first area and a second area, with the wound covering material 1 being attached to the first area of the carrier material and the second area of the carrier material being at least partially self-adhesive. Such a carrier material can be a self-adhesive patch.

Especially it can be provided that the wound covering is permeable to air. Therefore the carrier material and the wound covering material 1 can be permeable to air. Advantageously the wound covering enables aeration of the affected area of the wound 2.

It can also be provided that the wound covering is impermeable to moisture. Therefore the carrier material and the wound covering material 1 can be impermeable to moisture. Advantageously the wound covering prevents drainage of the wound 2.

## Claims

1. Wound covering material (1) for directly contacting a wound (2) comprising a fabric (3) made of fibers (4), **characterized in that** the fibers (4) are natural hydrophobic hollow fibers (4).

2. Wound covering material (1) according to claim 1, wherein the fabric (3) is a nonwoven fabric, woven fabric, knitted fabric or net.

3. Wound covering material (1) according to claim 1 or 2, wherein the natural hydrophobic hollow fibers (4) are covered with natural waxy layers (5).

4. Wound covering material (1) according to any of claims 1 to 3, wherein the natural hydrophobic hollow fibers (4) are poplar seed fibers (4) and/or kapok fibers (4).

5. Wound covering material (1) according to any of claims 1 to 4, wherein the natural hydrophobic hollow fibers (4) comprises an inner wall surface (6) and a outer wall surface (7), and that antimicrobial nanoparticles (8) is attached to the inner wall surface (6) and/or a outer wall surface (7).

6. Wound covering material (1) according to claim 5, wherein the antimicrobial nanoparticles (8) are metal oxides, in particular MoO₃, ZnO or MgO, or nano silver.

7. Wound covering comprising the wound covering material (1) according to claim 1 to 6.

8. Wound covering according to claim 7, wherein a carrier material comprises a first side with a first area and a second area, with the wound covering material (1) being attached to the first area of the carrier material and the second area of the carrier material being at least partially self-adhesive.

9. Wound covering according to claim 7 or 8, wherein the wound covering is permeable to air.

10. Wound covering according to any of claims 7 to 9, wherein the wound covering is impermeable to moisture.

11. Use of the wound covering material (1) according to any of claims 1 to 6 for covering a, particularly infected, wound (2).

12. Method for producing a wound covering material (1), comprising the steps:
- providing a mixture of raw natural hydrophobic hollow fibers (4) and a liquid solution,
- evenly distributing the natural hydrophobic hollow fibers (4) in the mixture,
- applying the mixture on a mechanical support, and
- drying the mixture.

13. Method according to claim 1, wherein a antimicrobial agent is added to the liquid solution or mixture, which antimicrobial agent forms antimicrobial nanoparticles (8) on inner wall surfaces (6) and/or outer wall surfaces (7) of the fibers (4) during the drying.
